# EUROPEAN PATENT SPECIFICATION

(11) **EP 1 357 843 B1**
(45) Date of publication and mention of the grant of the patent: **20.05.2009**
(21) Application number: 02718934.9
(22) Date of filing: 05.02.2002
(51) Int. Cl.: A61B 17/00, A61M 25/00

(54) **METHOD AND SYSTEM FOR TISSUE REPAIR USING DUAL CATHETERS**
VERFAHREN UND SYSTEM ZUR GEWEBEREPARATUR MITTELS ZWEIER KATHETER
METHODE ET SYSTEME DE REPARATION TISSULAIRE AU MOYEN DE CATHETERS DOUBLES

(30) Priority: 06.02.2001 US 778392
(43) Date of publication of application: 05.11.2003
(73) Proprietor: Edwards Lifesciences Corporation, Irvine, CA 92614 (US)
(72) Inventor: SHRECK, Stefan, G., Vista, CA 92083 (US); ALLEN, William, J., Stratford, CT 06497 (US); REED, Scott, Monroe, CT 06468 (US); BACHMAN, Alan, B., New Haven, CT 06513 (US); STECKEL, Robert, R., Norwalk, CT 06854 (US); FEDERICK, Karl, T., Bethel, CT 06801 (US); ADAMS, Leland, R., Ansonia, CT 06401 (US); CHAPOLINI, Robert, Phoneix, MD 21131 (US)
(74) Representative: Parry, Christopher Stephen
(86) International application number: PCT/US2002/003835
(87) International publication number: WO 2002/062236

(56) References cited:
- US-A- 5 267 958
- US-A- 5 695 457
- US-A- 6 029 671

## Description

### FIELD OF THE INVENTION

The present invention relates to the repair of tissue, and, more particularly, to apparatus for the repair of tissue within the body of a patient by using a dual catheter system to stabilize the tissue, and if required, fasten the tissue portions together.

### BACKGROUND OF THE INVENTION

In vertebrate animals, the heart is a hollow muscular organ having four plumping chambers. The left and right atria and the left and right ventricles, each provided with its own one-way outflow valve. The natural heart valves are identified as the aortic, mitral (or bicuspid), tricuspid and pulmonary valves. The valves separate the chambers of the heart, and are each mounted in an annulus therebetween. The annuluses comprise dense fibrous rings attached either directly or indirectly to the atrial and ventricular muscle fibers. The leaflets are flexible collagenous structures that are attached to and extend inward from the annuluses to meet at coapting edges. The aortic and tricuspid valves have three leaflets, while the mitral and pulmonary valves have two.

Various problems can develop with heart valves, for a number of clinical reasons. Stenosis in heart valves is a condition in which the valves do not open properly. Insufficiency is a condition which a valve does not close properly repair or replacement of the aortic or mitral valves are most common because they reside in the left side of the heart where pressures and stresses are the greatest. In a valve replacement operation, the damaged leaflets are excised and the annulus sculpted to receive a replacement prosthetic valve.

In many patients who suffer from valve dysfunction, surgical repair (i.e., "valvuloplasty") is a desirable alternative to valve replacement. Remodeling of the valve annulus (i.e., "annuloplasty") is central to many reconstructive valvuloplasty procedures. Remodeling of the valve annulus is typically accomplished by implantation of a prosthetic ring (i.e. "annuloplasty ring") to stabilize the annulus and to correct or prevent valvular insufficiency that may result from a dysfunction of the valve annulus. Annuloplasty rings are typically constructed of a resilient core covered with a fabric sewing ring. Annuloplasty procedures are performed not only to repair damaged or diseased annuli, but also in conjunction with other procedures, such as leaflet repair.

Mitral valve regurgitation is caused by dysfunction of the mitral valve structure, or direct injury to the mitral valve leaflets. A less than perfect understanding of the disease process leading to mitral valve regurgitation complicates selection of the appropriate repair technique. Though implantation of an annuloplasty ring, typically around the posterior aspect of the mitral valve, has proven successful in a number of cases, shaping the surrounding annulus does not always lead to optimum coaptation of the leaflets.

More recently, a technique known as a "bow-tie" repair has been advocated. The bow-tie technique involves suturing the anterior and posterior leaflets together in the middle, causing blood to flow through the two side openings thus formed. This technique was originally developed by Dr. Ottavio Alfieri, and involved placing the patient on extracorporeal bypass in order to access and suture the mitral valve leaflets.

A method for performing the bow-tie technique without the need for bypass has been proposed by Dr. Mehmet Oz, of Columbia University. The method and a device for performing the method are disclosed in PCT publication WO 99/00059, dated January 7, 1999. In one embodiment, the device consists of a forceps-like grasper device that can be passed through a sealed aperture in the apex of the left ventricle. The two mitral valve leaflets meet and curve into the left ventricular cavity at their mating edges, and are thus easy to grasp from inside the ventricle. The mating leaflet edges are grasped from the ventricular side and held together, and various devices such as staples are utilized to fasten them together. The teeth of the grasper device are linearly slidable with respect to one another so as to align the mitral valve leaflets prior to fastening. As the procedure is done on a beating heart, and the pressures and motions within the left ventricle are severe, the procedure is thus rendered fairly skill-intensive.

There is presently a need for an improved means for performing the bow-tie technique of mitral valve repair, preferably utilizing a minimally invasive technique.

US-A-6,029,671 discloses a guidewire; a probe having a distal portion, said probe comprising at least one guidewire lumen, said guidewire lumpen terminating in at least one guidewire port; said probe further comprising at least one additional lumen.

### SUMMARY OF THE INVENTION

The invention provides a system for performing a surgical procedure within a blood vessel, comprising: at least one guidewire, said guidewire adapted to be inserted into a body vessel; an antegrade probe having a distal portion, said antegrade probe comprising at least one antegrade guidewire lumen, said antegrade guidewire lumen terminating in at least one antegrade guidewire port; a retrograde probe having a distal portion, said retrograde probe comprising at least one retrograde guidewire lumen, said retrograde guidewire lumen terminating in at least one retrograde guidewire port, said at least one retrograde guidewire port co-aligned with said antegrade probe; and at least one of said antegrade probe and said retrograde probe further comprising at least one lumen in addition to said retrograde and antegrade guidewire lumens; wherein said antegrade probe and said retrograde probe are placed, in use, over said guidewire so that said guidewire resides within said at least one antegrade guidewire port and said at least one retrograde guidewire port and wherein said at least one retrograde guidewire port is aligned with said at least one antegrade guidewire port.

The present invention provides a system for approximating tissue using at least two catheters. More particularly, the present invention discloses a system of approximating a number of devices for stabilizing tissue and fastening or "approximating" a single portion or discrete pieces of tissue through the use of at least two probes directed to the area of interest by at least one guidewire. The tissue of interest may be straight, curved, tubular, etc. For example, many of the embodiments of the invention disclosed herein are especially useful for joining two leaflets of a heart valve. The coapting edges of the leaflets thus constitute the "tissue pieces." In other contexts, the invention can be used to repair arterial septal defects (ASD), ventricular septal defects (VSD), and in cases involving patent foraman ovale. Additionally, the present invention may be used during valve replacement surgery, to deploy a plurality of valve repair devices. In sum, the present invention in its broadest sense should not be construed to be limited to any particular tissue pieces, although particular examples may be shown and disclosed.

The following description discloses a number of guidewire-directed devices for both stabilizing the tissue pieces to be joined, and fastening them together. Some embodiments disclose only the stabilizing function, others only the fastening function, and still other show combinations of stabilizing and fastening devices. It should be understood that certain of the stabilizing devices may be used with certain of the fastening devices, even though they are not explicitly shown in joint operation. In other words, based on the explanation of the particular device, one of skill in the art should have little trouble combining the features of certain of such devices. Therefore, it should be understood that many of the stabilizing and fastening devices are interchangeable.

Furthermore, many of the fastening devices disclosed herein can be deployed separately from many of the stabilizing devices, and the two can therefore be deployed in parallel.

The guidewire-directed stabilizing and fastening devices of the present invention can be utilized, for example, in endoscopic procedures, beating heart procedures, or percutaneous procedures. In yet another embodiment the devices can be delivered into the heart through the chest via a thorascope. The devices can also be delivered percutaneously, via a catheter or catheters, into the patient's arterial system (e.g. through the femoral or brachial arteries). Other objects, features, and advantages of the present invention will become apparent from a consideration of the following detailed description.

Other objects, features, and advantages of the present invention will become apparent from a consideration of the following detailed description.

### Brief Description of the Drawings

Figure 1 is a elevational view of a step in a valve repair procedure using the present invention;
Figure 1a is an elevational view of an embodiment of a vacuum based probe of the present invention;
Figure 1b is an elevational view of an embodiment of a vacuum based probe of the present invention disposing including vanes;
Figure 2 is an elevational view of an embodiment of a vacuum based probe of the present invention having a tapered nose and disposing vanes;
Figure 2a is an sectional view of a step in a valve repair procedure using the tissue stabilizer of Figure 2;
Figures 3a-3c are perspective views of several embodiments of vacuum-based tissue stabilizers having tissue separating walls;
Figures 3d and 3e are sectional views of two different vacuum port configurations for the tissue stabilizers shown in Figures 3a-3c, the stabilizers shown in operation;
Figure 4a is an elevational view of a first step in a valve repair procedure using a mechanical tissue stabilizer with linearly displaceable tissue clamps;
Figure 4b is an elevational view of a second step in a valve repair procedure using the tissue stabilizer of Figure 4a;
Figure 4c is a detailed perspective view of a clamp of the tissue stabilizer of Figure 4a extended to grasp a valve leaflet from both sides;
Figure 5a is a perspective view of a suture-based tissue fastener of the present invention having toggles;
Figure 5b is a sectional view of the suture-based tissue fastener of Figure 5a loaded into a delivery needle;
Figures 6a-6c are elevational views of several steps in a valve repair procedure using a tissue stabilizer of the present invention and the suture-based tissue fastener shown in Figure 5a.
Figure 7 is an elevational view of an alternative tissue stabilizing and fastening device;
Figures 8a-8c are sectional views of a tissue stabilizing and fastening device of the present invention having needles deployed by the retrograde probe on the ventricular side of the tissue being received by the antegrade probe;
Figure 9a is a perspective of a further tissue fastening device of the present invention comprising a staple-like tissue fastener in an open configuration;
Figure 9b is a perspective view of further tissue fastening device of the present invention comprising a staple-like tissue fastener in a closed configuration;
Figures 10a-10c are sectional views of several steps in a valve repair procedure using an exemplary tissue fastening device of the present invention for delivering the tissue staple of Figures 9a-9b;
Figure 11 is a perspective view of a completed valve repair procedure utilizing the tissue stabilizing and fastening device of Figures 10a-10c;
Figure 12 is an elevational view of an alignment mechanism of the present invention of the present invention;
Figures 13a-13b are sectional views of a wire-based steering mechanisms of the present invention;
Figures 14a-14b are sectional view of the steering sleeve based steering mechanism of the present invention;
Figure 15 is a sectional view of the steering balloon based steering mechanism of the present invention; and
Figures 16a-16c are sectional views of several steps in a tissue repair procedure using an exemplary sequential tissue repair device of the present.

### DETAILED DESCRIPTION OF THE INVENTION

The system of the present invention is designed for use in the surgical treatment of bodily tissue. As those skilled in the art will appreciate, the exemplary guidewire-directed dual catheter tissue repair system disclosed herein is designed to minimize trauma to the patient before, during, and subsequent to the surgical procedure, while providing improved device placement and enhanced tissue stabilization. Additionally, the guidewire-directed dual catheter tissue repair system, by utilizing two separate and distinct probes that cooperatively interact, may be adapted to precisely deliver and deploy a plurality of tissue fasteners to an area of interest. For example, the present system may be utilized to repair mitral valve tissue by stabilizing the discrete tissue pieces and deploying a fastening device thereby coapting the tissue pieces. As those skilled in the art will appreciate, the present invention may similarly used to repair Arterial Septal Defects (ASD), Ventricular Septal Defects (VSD), and defects associated with Patent Foramen Ovale (PFO).

Disclosed herein is a detailed description of various illustrated embodiments of the invention. This description is not to be taken in a limiting sense, but is made merely for the purpose of illustrating the general principles of the invention. The section titles and overall organization of the present detailed description are for the purpose of convenience only and are not intended to limit the present invention.

As those skilled in the art will appreciate, the present invention permits the operator to position at least two guidewire-directed probes within a body vessel and utilize the cooperative effects of the two positions and deploy a plurality of fastening devices to surrounding tissue. In the illustrated embodiment, the two probes comprise an antegrade probe positioned proximate to the superior or atrial portion of the mitral valve, and a retrograde probe positioned proximate to the inferior or ventricular portion of the mitral valve. It is anticipated as being within the scope of the present invention to utilize the present invention to perform a plurality of surgical procedures, and may deliver and deploy a plurality of tissue fastening devices to an intra-vascular area.

For example, the present device may be utilized to repair defects in the arterial septum. At least two guidewire-directed probes, one probe addressing the tissue from an antegrade position and the other probe addressing the tissue from a retrograde position, are used to stabilize the arterial septal tissue. Once stabilized, a fastening device maybe deployed to repair the defect. Similarly, the present invention maybe used to repair venticular septal defects, or defects relating to patent foramen ovale.

### A. Exemplary Procedure Description

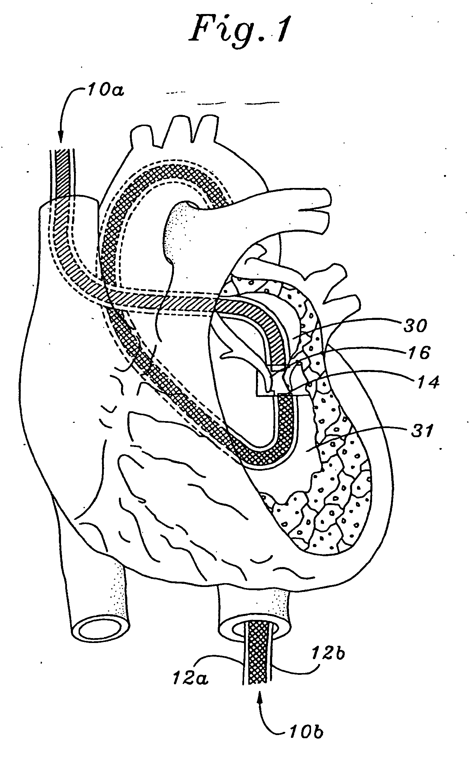

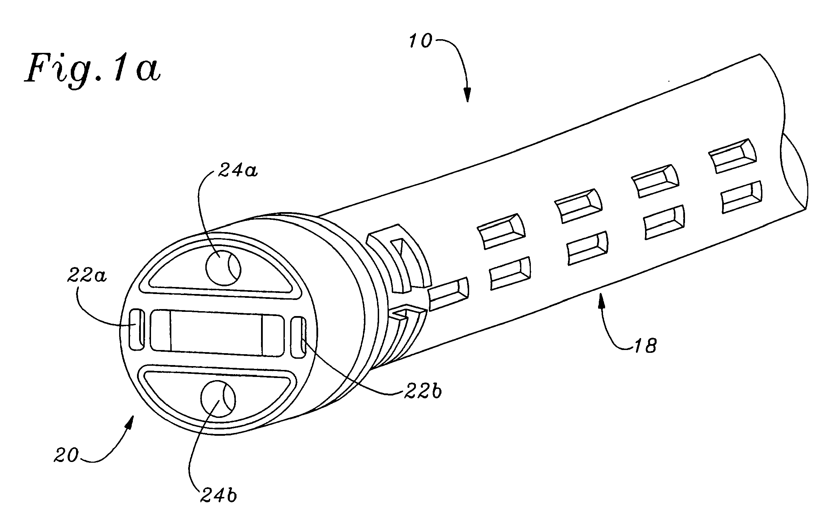

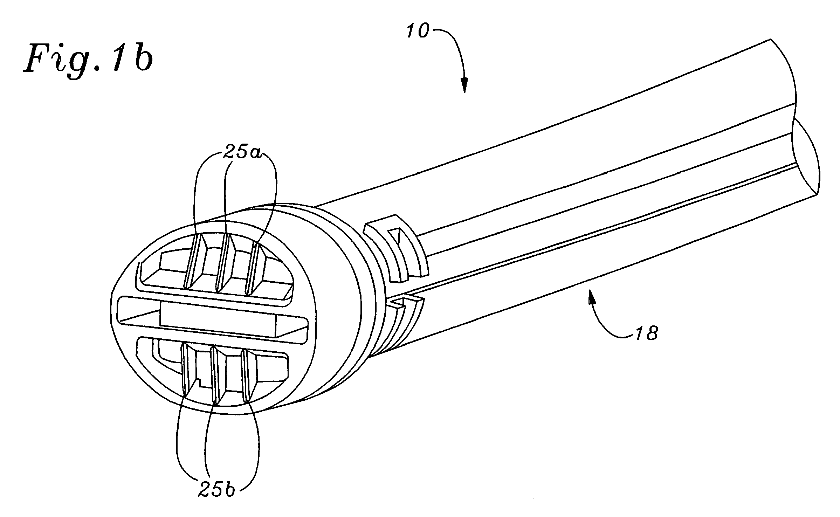

**Figure 1** shows an embodiment of the present invention being utilized to repair a heart valve. More particularly, **Figure 1** shows a guidewire-directed antegrade probe **10a** and retrograde probe **10b** being used to stabilize and repair the tissue leaflets **14** and **16** of the mitral valve.

A first guidewire **12a,** capable of traversing the circulatory system and entering the heart, is introduced into the femoral vein of a patient (or, alternatively the right jugular vein) through an endoluminal entry point. The first guidewire **12a** is advanced through the circulatory system eventually arriving at the heart. Upon arriving a the heart, the first guidewire **12a** enters the right atrium of the heart. The first guidewire **12a** is directed to traverse the right atrium and puncture the atrial septum, thereby entering the left atrium. The first guidewire **12a** is progressed through the mitral valve while the heart is in diastole thereby entering into the left ventricle. Thereafter the first guide wire **12a** is made to traverse through the aortic valve into the aorta and is made to emerge at the left femoral artery through a endoluminal exit point. This methodology is known to physicians skilled in interventional cardiology. Once first guide wire **12a** is positioned, a second guide wire **12b** similarly traverses the circulatory system and is positioned proximal to first guide wire **12a** using techniques familiar to those skilled in the art. The endoluminal entry and exit ports are dilated to permit entry of at least one probe. A protective sheath may be advanced within the venous area to protect the inner venular structure.

With guidewires **12a** and **12b** suitably anchored, the antegrade probe **10a** is attached to the guidewires **12a** and **12b** and advanced through the dilated guide wire entry point to a point proximal to the arterial cusp portion of the mitral valve. The distal portion of antegrade probe **10a,** having at least one vacuum port in communication with at least one vacuum lumen contained within at least one internal lumen of the probe, is positioned proximate the tissue leaflets **14** and **16** of the mitral valve. Once positioned, the antegrade probe **10a** may use vacuum force to capture and grasp the mitral tissue, grasp the tissue and deploy a fastening device, grasp and manipulate the mitral tissue, or grasp and manipulate the tissue to a desired positioned and deploy a fastening device. The manipulation or steering of the mitral tissue is accomplished by positioning the at least one vacuum port proximate the mitral tissue and activating the vacuum source. The mitral tissue will be forcibly retained by the vacuum force, thereby permitting the operator to steer or position tissue.

A retrograde probe **10b** is attached to at least one guidewire and introduced into the body through dilated guidewire exit point. The flexible retrograde probe **10b** is advanced through the body vessel, entering the heart through the aortic valve and progressing into the left ventricle. The distal portion of retrograde probe **10b** is proximal the ventricular portion of the of the mitral valve. The retrograde probe **10b** may include a distal portion having at least one vacuum port connected to at least vacuum lumen contained within at least one internal lumen, thereby permitting retrograde stabilization of tissue.

With the antegrade probe and retrograde probe suitably positioned, the external vacuum source connected to the antegrade probe, retrograde probe, or both, is activated, thereby permitting mechanical capture of the tissue. Upon successful tissue capture, a detachable fastening device mechanically retained either by antegrade probe **10a** or retrograde probe **10b,** or both, is forcibly deployed piercing the valve tissue and thereby mechanically joining the cusps of the mitral valve. These fastening devices may include self-closing fasteners, spring loaded fasteners, pre-formed fasteners, latching fasteners, and rotatably deployed fasteners.

To complete the procedure, the external vacuum source is deactivated, resulting in tissue release. The two probes are retracted through their individual entry points, and the two guidewires are removed. Finally, the endoluminary entry point and exit point are sutured.

### B. Exemplary Guidewire Devices

**Figure 1** shows a guidewire-directed dual catheter tissue stabilizer system comprising an antegrade probe **10a** and a retrograde probe **10b** of the present invention that is used to stabilize two tissue pieces **14** and **16;** respectively. The guidewires **12a** and **12b** may be formed of a single filament or a multi-filament wound system, and may be comprised of materials known to those skilled in the art of minimally invasive surgery, including, without limitation, a Nickel- Titanium (Ni Ti) compound, stainless steel #304, 304V, 312, and 316, or other suitable material. Likewise, the guidewires may be coated with a biologically-compatible lubricant or with a biologically-compatible sealant such as polytetrafluoroethylene (PTFE). The guidewires should have sufficient structural flexibility and steerability to permit intraluminal positioning, while retaining sufficient structural integrity to position tissue stabilizers. Additionally, the guidewires may have a substantially circular profile, or, alternatively, may be shaped to provide a degree of axial control. For example, a wire incorporating a substantially octagonal profile would provide sufficient axial force to permit axial movement of the catheters along an axial arc.

During a procedure, a guidewire **12a** may be introduced to a body vessel in a plurality of manners, including, for example and without limitation, percutaneously, transapically, transatrially, or through a surgical incision proximate the area of interest. Guidewire **12a** is then positioned proximate to or traversing the area of interest. Once positioned and sufficiently anchored, a second guidewire **12b** may be similarly introduced to traverse the pathway established by guidewire **12a,** and likewise positioned within the mitral valve and suitably anchored. It should be understood that the present invention contemplates without limitation either a single guidewire or multiple guidewire approach. These guidwire or guidewires will direct and precisely position probes **10a** and **10b** proximate the area of interest. Upon completion of the procedure, the probes **10a** and **10b** and the guidewire (not shown) or guidewires **12a** and **12b** are removed from the body vessel.

### C. Exemplary Tissue Stabilizing Devices

It should be understood that the antegrade and the retrograde probe disclosed herein cooperatively interact to provide stabilizing force to the tissue interposed therebetween. For example, the cooperative interaction may consist of the application of force to opposing surfaces of tissue interposed between the probes, vacuum force applied by either or both probes, and mechanical retaining devices, as detailed below, disposed on either or both probes. It is understood that both probes utilize at least one guidewire slidably attached to the distal portion of each probe to precisely position and align the probes. Furthermore, it is understood that the antegrade probe or the retrograde probe, or both, may apply the retentive force to stabilize tissue. Additionally, tissue fastening device may be disposed about the proximal portion of the antegrade probe or the retrograde probe, or both, to approximate two pieces of tissue disposed between the opposing probes. A deployable alignment mechanism may be disposed about the distal portion of the antegrade probe or retrograde probe, or both, thereby ensuring a precise positioning of either or both probes with relation to the tissue.

**Figure 1** shows two probes **10a** and **10b** of the present invention that uses a vacuum to stabilize two tissue pieces **14** and **16,** respectively. In this case, the procedure being conducted is a repair of a heart valve using an arterial probe **10a** and a ventricular probe **10b.** The at least two probes **10a** and **10b** may share common elements and will be generically described as probe **10.**

As shown in **Figure 1a****,** the probe **10** comprises a cylindrical probe body **18** with at least one internal lumen (not shown) and having a flat distal portion **20** disposing at least two guidewire ports, **22a** and **22b,** and at least two vacuum ports **24a** and **24b.** It should be noted that the illustrated embodiment utilizes two guidwires, though the system may be operated using a single guidewire. The at least two guidewire ports, **22a** and **22b,** which are connected to at least two guidewire lumens (not shown), are disposed radially about the distal portion **20** of the probe **10,** and are substantially parallel to the longitudinal axis of at least one internal lumen (not shown). The at least two vacuum ports **24a** and **24b,** are in communication with an external vacuum source through the at least one internal lumen (not shown). The size of the ports, namely **24a** and **24b,** and magnitude of suction applied may be vary depending on the application. The spacing between the ports **24a** and **24b** should be sufficiently spaced so as to create independent suction regions. In this manner, one leaflet or the other may be stabilized with one of the ports, e.g. **24a,** without unduly influencing the other port, e.g. **24b.** In one example, the ports **24a** and **24b** have a minimum diameter of about 3,175 mm (1/8 inch), and are spaced apart with a wall of at least 0,508 mm (0.020 inches) therebetween.

As shown in **Figure 1b****,** the distal portion **20** may dispose a series of vanes, **25a** and **25b,** positioned proximate the vacuum ports **24a** and **24b.** The vane series, **25a** and **25b,** respectively, may be recessed from the distal portion **20,** thereby forming a tissue supporting structure when vacuum force is applied to pliable tissue. Preferably, the vanes **25a** and **25b** are recessed approximately 0,0508 to 0,254 mm (0.002 to .01 inches) from the distal portion **20.**

The probe **10** desirably has a size suitable for minimally invasive surgery. In one embodiment probe **10** is part of a catheter based percutaneous delivery system. In that case probe **10** is a catheter tube having one or more lumens connecting vacuum ports **24a** and **24b** to the vacuum source or sources. The catheter would be long enough and have sufficient steerability and maneuverability to reach the heart valve from a peripheral insertion site, such as the femoral or brachial artery. One particular advantage of the present invention is the ability to perform valve repair surgery on a beating heart.

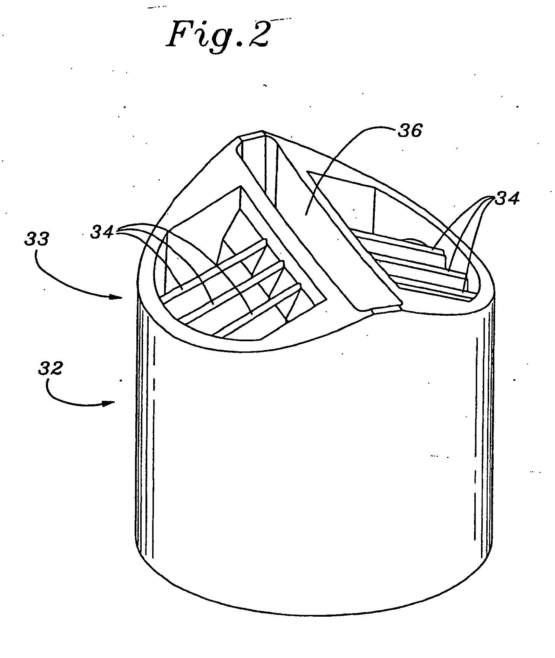

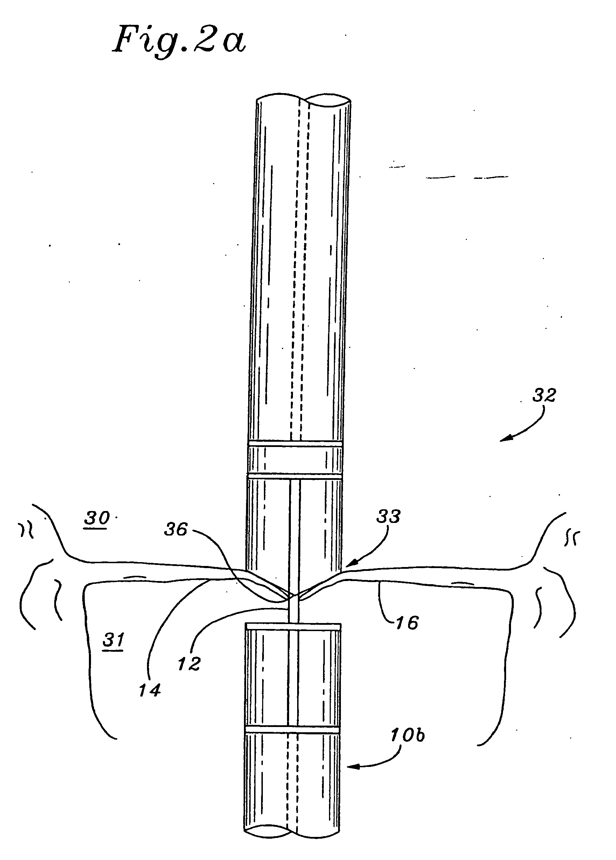

**Figure 2** illustrates an additional embodiment of the present invention utilizing a tapered distal portion of the probe. The probe **32** has a distal potion **33** which includes a series of recessed vanes **34** connected to at least one internal lumen (not shown) to stabilize tissue. An additional port **36** may be used to deploy or receive a plurality of fastening devices.

**Figure 2a** shows an illustrative valve repair procedure using the probe **32** of **Figure 2** approching the tissue from the arterial side **30** of the valve, while additionally stabilizing the tissue with probe **10b** from the ventricular side **31** of the valve. The port **36** at the distal tip of the nose is exposed to the ventricular **31** side of the leaflets **14** and **16.** Because of this exposure, various leaflet fastening devices can be delivered through the probe **32** to the ventricular **31** side of the leaflets **14** and **16,** as will be detailed below. Likewise, a tissue fastening device may be deployed by probe **10b** through the leaflets, **14** and **16,** to the probe **32** positioned proximal to the arterial portion of the mitral valve. Interference with the stabilization process by guidewire **12** is negligible. Those skilled in the art will appreciate either the antegrade probe, the retrograde probe, or both, may utilize the tapered nose design detailed herein.

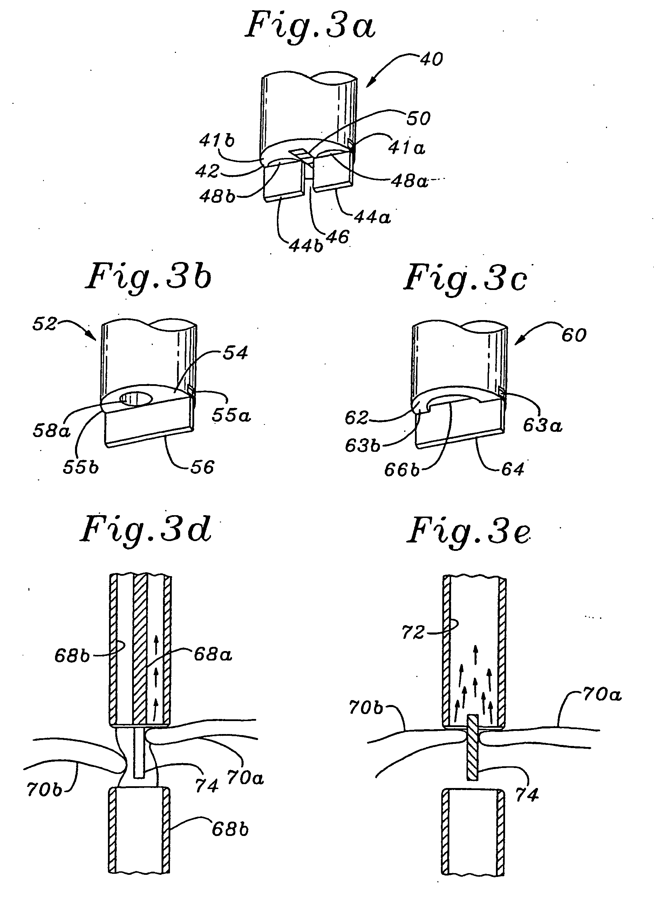

**Figures 3a-3c** show three vacuum-based tissue stabilizing probes having tissue separating walls. In **Figure 3a**, a tissue stabilizer **40** includes at least two guidewire ports **41a** and **41b** radially about the distal portion of the probe, having a flat distal face **42** having a pair of distally-directed tissue separating walls **44a** and **44b** extending therefrom, and defining a gap **46** therebetween. The stabilizer **40** contains one or more lumens in communication with vacuum ports **48a** and **48b,** that open on both sides of the walls **44a** and **44b.** In addition, a fastener channel **50** opens at the distal face **42** between the walls **44a** and **44b,** and facing the gap **46** therebetween. The fastener channel **50** can be used to deliver tissue fasteners, as described below.

In **Figure 3b****,** a tissue stabilizer **52** includes a flat distal face **54** disposing at least two guidewire ports **55a** and **55b,** and having a single distally-directed tissue separating wall **56** extending therefrom. The stabilizer **52** contains one or more lumens in communication with circular vacuum ports **58a** and **58b** (not shown) that open on either side of the wall **56.**

In **Figure 3c****,** a tissue stabilizer **60** includes a flat distal face **62,** disposing at least two guidewire ports **63a** and **63b** radially positioned about distal face **62,** and having a single distally-directed tissue separating wall **64** extending therefrom. The stabilizer **60** contains one or more lumens in communication with semi-circular vacuum ports **66a** (not shown) and **66b** that open on both sides of the wall **64.** There are two such ports **66a** (not shown) and **66b,** one on each side of each wall **64.**

**Figures 3d** and **3e** show two different vacuum port configurations for the tissue stabilizers **40, 52,** or **60** shown in **Figures 3a-3c****.** As mentioned above, the stabilizers **40, 52,** or **60** may have one or more lumens in communication with one or more ports. In Figure **3d****,** two lumens **68a** and **68b** provide separate suction control to the associated ports. Thus, one tissue piece **70a** is seen stabilized by the righthand vacuum port, while the left-hand port is not operated. Alternatively, a single lumen **72** in communication with two vacuum ports is seen in **Figure 3e****,** and both tissue pieces **70a, 70b** are stabilized simultaneously. In both these views, the tissue separating wall **74** is shown between the tissue pieces to be joined. Fastening devices can thus be delivered via the wall **74,** or through a gap formed for that purpose, such as the gap **46** and fastener channel **50** seen in **Figure 3a****.**

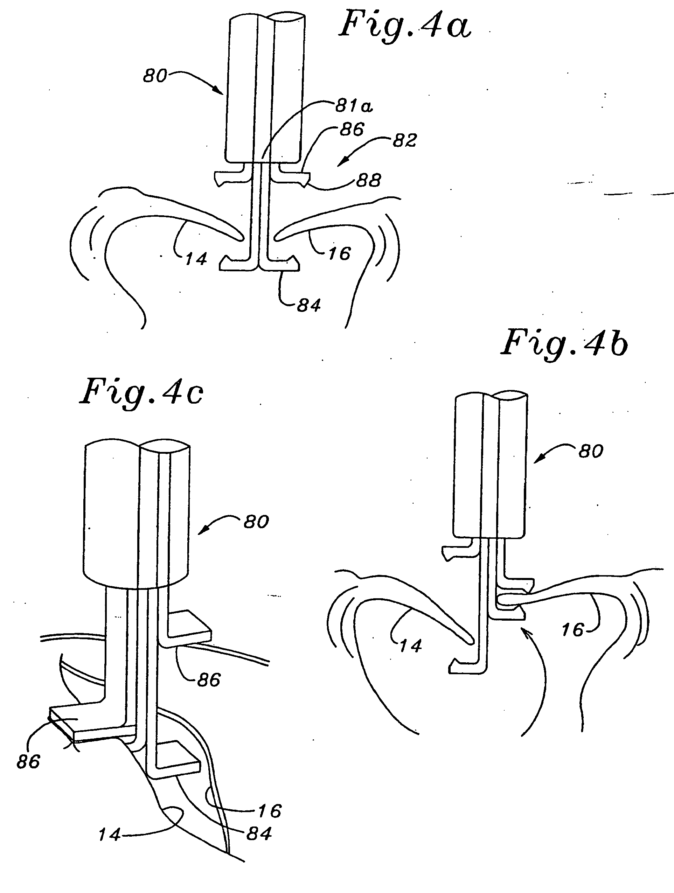

**Figures 4a-4c** show a mechanical tissue stabilizer **80** with a four-part, linearly displaceable tissue clamp **82,** disposing at least two guidewire ports 81 **a** and **81b** (not shown), respectively, positioned radially about the distal portion of the stabilizer **80.** On each side, a lower clamp **84** is separated from an upper clamp **86** and inserted between two tissue pieces (in this case valve leaflets **14** and **16**). As the lower and upper clamps **84, 86** are brought together, as seen in **Figure 4b****,** they physically clamp and stabilize the leaflet **16.** Small teeth **88** on the clamps **84** and **86** may be provided for traction. The clamps **84** and **86** on each side are individually actuated to enable grasping of one leaflet at a time. Once the tissue has been suitably captured by antegrade probe **80** an retrograde probe (not shown) is utilized to deploy a fastening device to the captured tissue.

As stated above, the dual catheter system disclosed herein contemplates utilizing the probes disclosed above in a cooperative manner. As those skilled in the art will appreciate, various arterial probes may be used with various ventricular probes, thereby providing a dual catheter system capable of customization dependant on need. For example, an arterial probe having a tapered nose may be used with a ventricular probe having a flat distal portion. Alternatively, an arterial probe having a flat distal portion may be utilized with a ventricular probe having a tapered nose. As those skilled in the art will appreciate the system may be easily tailored accordingly.

### D. Exemplary Tissue Fasteners

As stated in the previous sections, the present invention contemplates using at least one guide wire to direct and position at least two co-operatively functioning probes to an area of interest. In a preferred embodiment, at least two probes, each disposing at least two guidewire ports proximate to the distal portion thereof, would be directed to an area of interest by at least two guidewires. It should be understood that the present invention discloses using at least two guidewire-directed probes simultaneously to perform a surgical therapeutic procedure. The following sections disclose exemplary tissue fasteners capable of deployment with the guidewire-directed dual catheter system of the present invention. The figures associated with the following sections are intended to illustrate novel fastening systems. As such, only one catheter may be illustrated, but a second catheter is assumed. Likewise, the following systems employ at least one guidewire and at least two guidewire ports disposed proximal the distal portion of the probes. To permit clear illustration of the novel fastening systems disclosed herein the guidewire or guidewire and guidewire ports may not be illustrated in the following figures, but should be assumed included.

### 1. Exemplary Suture-Based Tissue Fasteners

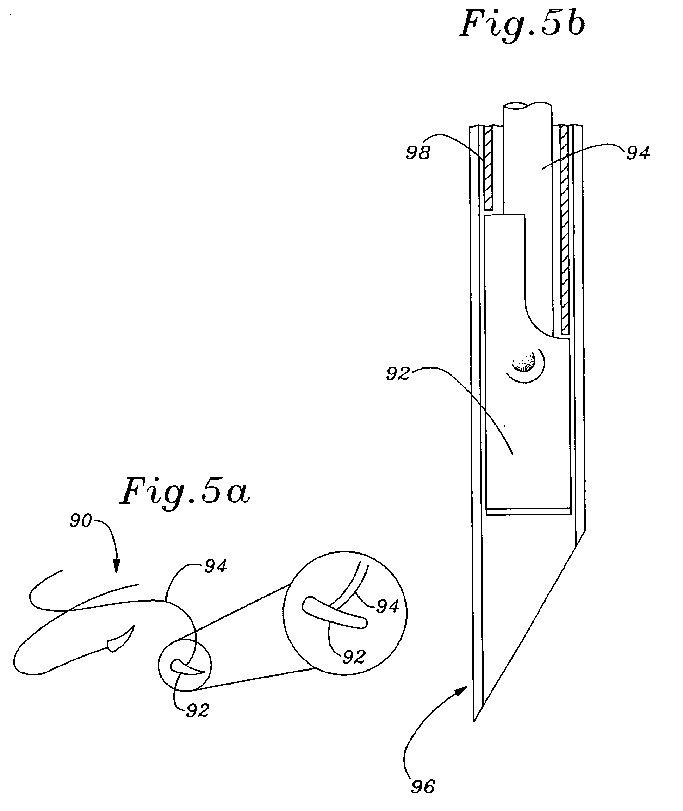

**Figure 5a** illustrates a suture-based tissue fastener **90** of the present invention including toggles **92** secured to the end of suture threads **94.** **Figure 5b** is a sectional view through a needle **96** used to deliver the tissue fastener **90.** Specifically, the toggle **92** and suture thread **94** is seen loaded into the lumen of the needle **96,** and a pusher **98** is provided to urge the tissue fastener **90** from the distal end thereof. The fastener **90** maybe deployed by the antegrade probe, the retrograde probe, or both.

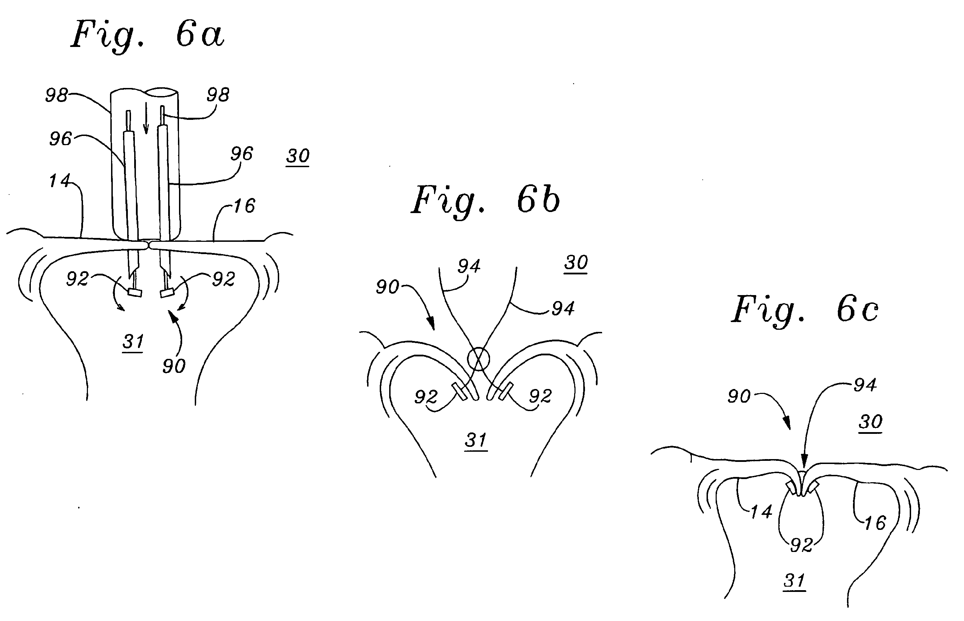

**Figures 6a-6c** depict several steps in a valve repair procedure using the tissue fasteners **90** shown in **Figure 5a****.** A probe, such as the probe **10** seen in **Figure 1** having vacuum ports for tissue stabilization and guidewire ports positioned radially about the distal portion of probe **10,** provides lumens for two of the needles **96** of **Figure 5b****.** The lumens with the vacuum ports may receive the needles **96** or additional lumens may be provided. The sharp ends of the needles **96** pierce the leaflets, and the pushers **98** are displaced (separately or in conjunction) to deploy the tissue fasteners **90.** After the needles **96** are retracted, the toggles **92** anchor the tissue fasteners **90** on the ventricular **31** side of the leaflets. The suture threads **94** are then tied off on the atrial **30** side to secure the leaflets **14** and **16** together, as seen in **Figure 6c**. The retrograde probe used to stabilize the tissue is not shown to permit clear illustration of the novel fastening device. As with all system disclosed herein, simultaneous use of an antegrade probe and retrograde probe is contemplated.

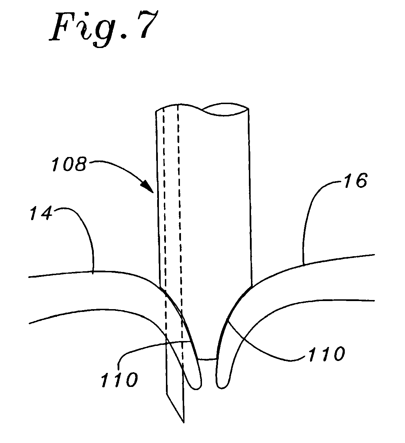

**Figure 7** illustrates an alternative tissue stabilizing and fastening device **108** having a pointed nose with two concave faces **110** in which the vacuum ports are located. The device **108** functions as described above, with a fastener deliver needle shown in phantom having pierced the left leaflet **14.** A retrograde probe (not shown) may be adapted to receive the fastening device **108** as well as stabilize the tissue.

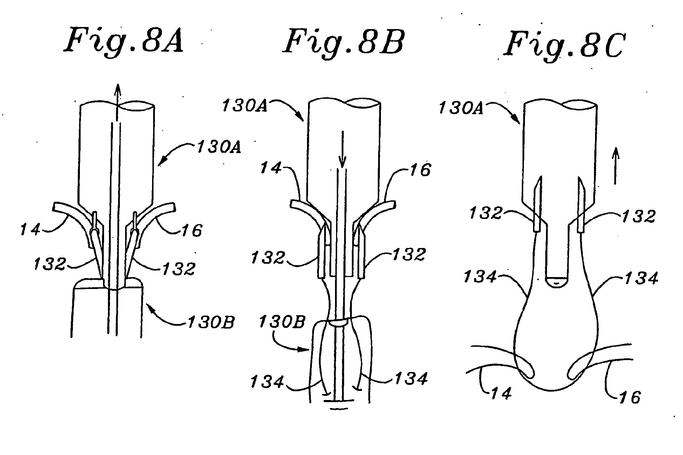

**Figures 8a-8c** illustrate a tissue stabilizing and fastening device **130a-b** having needles **132** deployable on a blind side of the tissue by the retrograde probe **130b.** A common suture thread **134** connects the needles **132** and is used to secure the tissue pieces **14** and **16** together. Thus, as seen in the sequence of **Figures 8a**-**8c,** the needles **132** are first advanced to a position proximate the tissue pieces **14** and **16** and deployed outboard of the distal tip of the retrograde probe 130b. Once positioned, the needles are advanced through the tissue, as in **Figure 8a****,** to cause the needles **132** to pierce the tissue pieces **14** and **16.** The two needles **132** are then disengaged from the device **130b,** and each other, as in **Figure 8b****,** and antegrade probe **130a** captures the needles **132** from the pieces **14** and **16,** leaving the connected suture joining the two pieces **14** and **16** (**Figure 8c**). The suture **132** can then be tied off, or otherwise secured on the upper side of the tissue pieces **14** and **16.**

### 2. Exemplary Staple and Clip-Type Fasteners

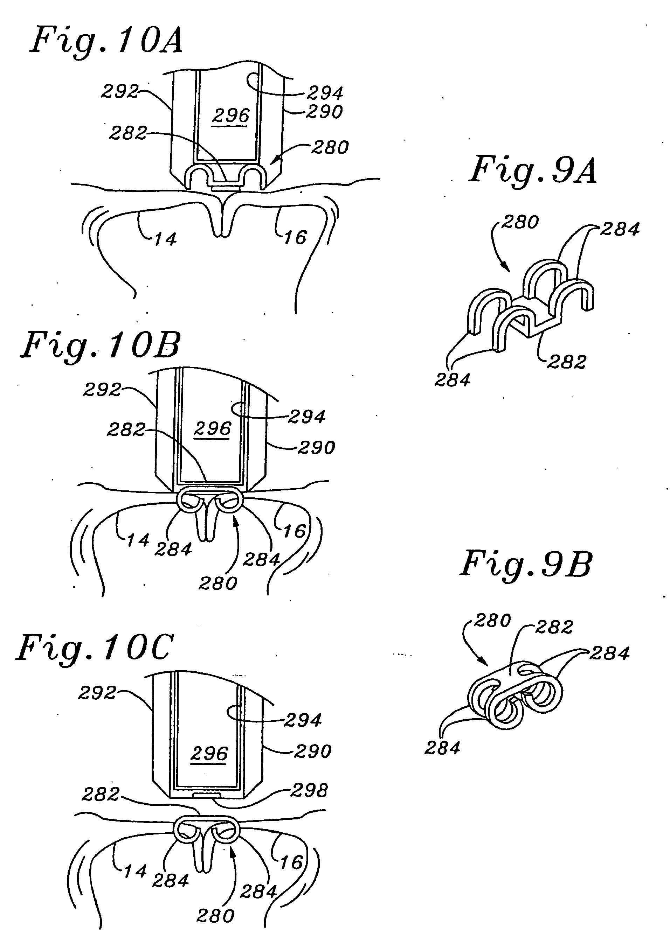

**Figure 9a** shows an exemplary tissue staple **280** for joining two tissue pieces in an open configuration. The staple **280** includes a bridge portion **282** and four gripping arms **284,** two on each side. The gripping arms **284** are initially curled in a semi-circle upward from the plane of the bridge portion **282** and terminate in sharp points approximately in the plane of the bridge portion **282.** **Figure 9b** shows the staple **280** when closed, with the gripping arms **284** curled underneath the plane of the bridge portion **282** toward each other.

**Figures 10a-10c** illustrate several steps in a valve repair procedure using an exemplary tissue fastening device **290** for delivering the tissue staple **280.** As with the previous embodiments, a retrograde probe (not shown) is utilized to stabilize the tissue prior to and during deployment of the fastening device. Additionally, the retrograde probe (not shown) may be used as an anvil or stop-body to assist in closing the fastener. The device **290** includes a probe **292** with an internal lumen **294** within which a pusher **296** is slidable, and having at least two guidewire ports (not shown) positioned radially about the distal portion of the probe. A stop member **298** is also provided underneath the bridge portion **282** of the staple **280** to prevent displacement of the bridge portion **282** toward the leaflets **14** and **16.** The probe is positioned proximate the tissue under repair. After stabilizing the leaflets **14** and **16,** the pusher **296** displaces downward which causes the staple **280** to undergo a plastic reformation from the configuration of **Figure 10a** to that of **Figure 10b****.** The sharp points of the gripping arms **284** pass through the leaflets **14** and **16** and anchor the staple **280** therein. Finally, the stop member **298** is disengaged from under the bridge portion **282,** and the device **290** is retracted.

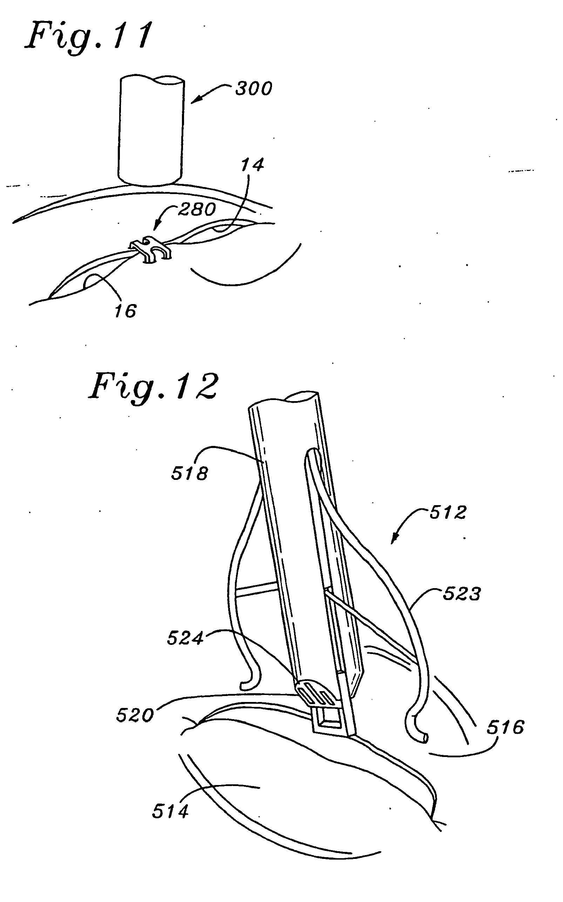

**Figure 11** illustrates the use of a tissue stabilizing and fastening device **300** for deploying the staple **280** of **Figure 9****.** The device **300** is quite similar to the device **290** of **Figure 10****,** with an exemplary stabilizing means shown in the form of vacuum chamber(s) **302** on each side of the staple deployment mechanism.

### E. Exemplary Probe Alignment Devices

An additional embodiment of the present invention includes, alignment mechanisms which may be affixed to the probe to precisely position at probe proximate within a body vessel. Those skilled in the art will appreciate the use of an alignment device in addition to the guidewire or guidewires disclosed above provides an inherently redundant alignment scheme, thereby permitting a more precise positioning of the probe relative to the area of interest.

**Figure 12** shows an antegrade probe of the antegrade and retrograde probe system of the present invention that uses a vacuum to hold two tissue pieces **514** and **516,** respectively. In this case, the tissue pieces are heart valve leaflets, **514** and **516,** and a valve repair procedure using an arterial probe **512a** and a ventricular probe **512b** (not shown). Probes **512a** and **512b** will hereinafter be generically described as probe **512.** As shown in **Figure 12****,** the probe **512** comprises a cylindrical probe body **518** with at least one internal lumen (not shown) and having a tapered distal portion **520** disposing at least one guidewire port (not shown) and at least one vacuum port **524.** At least one deployable alignment mechanism **523** is positioned proximate the probe distal portion **520** and is in communication with the handpiece (not shown) by a deployment conduit (not shown) positioned in at least one internal lumen (not shown) contained within probe **512.** Once the probe **512** is positioned proximate to the tissue **514** and **516,** respectively, the deployable alignment mechanism **523** is deployed and interacts with the surrounding tissue. The external vacuum source (not shown) is then activated. The at least one vacuum port **524** stabilizes tissue pieces **514** and **516.** Upon completion of the procedure, the deployable alignment mechanism **523** is retracted to facilitate removal of the probe **512.** While **Figure 12** shows the deployable alignment mechanism disposed on an antegrade probe, either the antegrade probe, retrograde probe, or both, may include deployable alignment devices.

### F. Exemplary Steering Devices

The present invention discloses a guidewire-directed system for repairing body tissue. Use of guidewire-directed flexible antegrade and retrograde catheters permits positioning of the devices proximal the tissue under repair. Locating the device proximate tissue under repair may be facilitated by supplemental steering mechanisms capable of permitting the probes to traverse acute angles. Several embodiments detailing a plurality of steering mechanisms are disclosed herein. The steering devices disclosed herein permit positioning of the antegrade catheter, retrograde catheter, or both, should supplemental steering mechanisms be required.

### 1. Steering Wire Approach

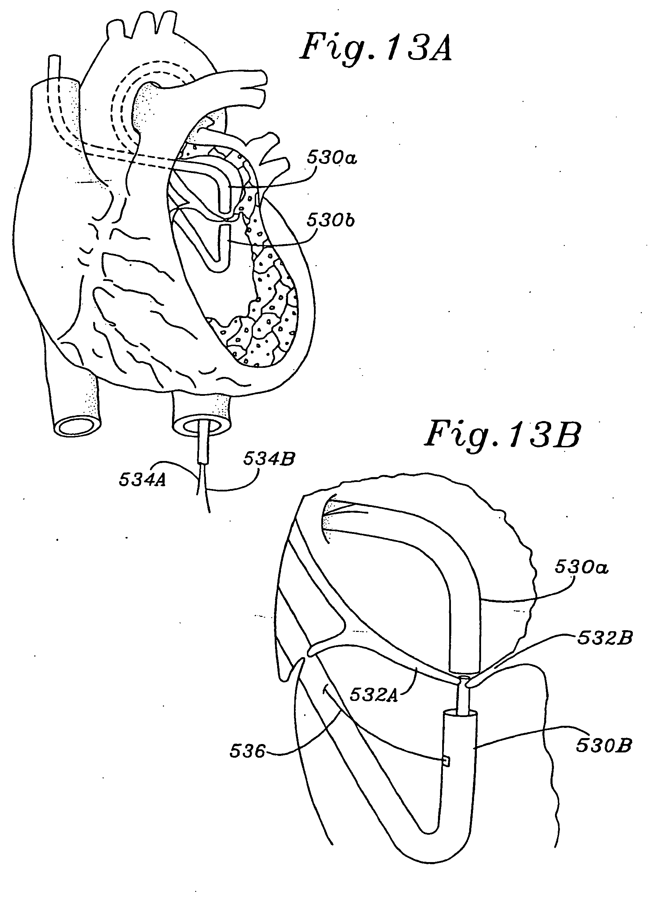

**Figures 13a-13b** show a mitral valve procedure being performed with the present invention. Antegrade probe **530a** is positioned proximate the arterial portion of the mitral tissue **532a** and **532b** by guidewires **534a** and **534b.** The retrograde probe **530b** is positioned proximate the ventricular portion of the mitral tissue **532a** and **532b,** and is similarly directed by guidewires **534a** and **534b.** Retrograde probe **530b** further disposes a steering conduit **536** which is connected to probe **530b** proximate the distal portion and which is in communication with the operator via at least one internal lumen (not shown) through a steering conduit port positioned on probe **530b.** The steering conduit **536** may be manufactured from a plurality of materials including a Nickel- Titanium (Ni Ti) compound, stainless steel #304, 304V, 312, and 316, or other suitable material.

### 2. Steering Sleeve Approach

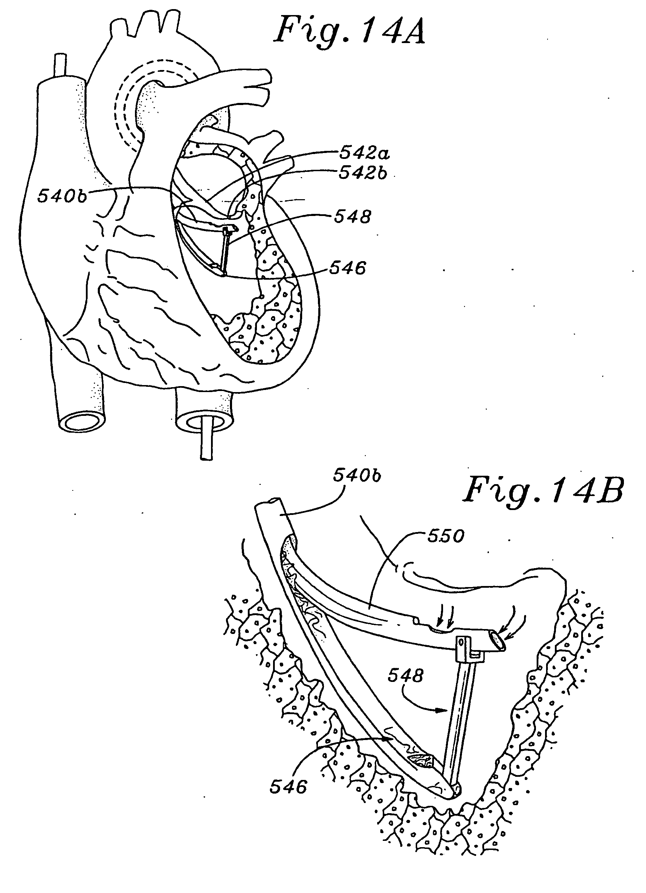

**Figures 14a-14b** show a mitral valve procedure being performed by the present invention. Antegrade probe (not shown) is positioned proximate the arterial portion of the mitral tissue **542a** and **542b** by guidewires (not shown). The retrograde probe **540b** is positioned proximate the ventricular portion of the mitral tissue **542a** and **542b,** and is similarly directed by the guidewires. Retrograde probe **540b** further disposes a steering sleeve **546** containing an actuated support **548** which is connected to a steering sleeve conduit **550** which is positioned within an internal lumen located in the probe **540b.** The probe **540b** and steering sleeve conduit 550 are positioned proximate the tissue under repair. Once positioned probe **540b** is advanced while the steering sleeve **546** is held stationary. Advancement of the probe **540b** results in extension of the actuated support **548** thereby positioning probe **540b** more proximate the tissue under repair.

### 3. Steering Balloon Approach

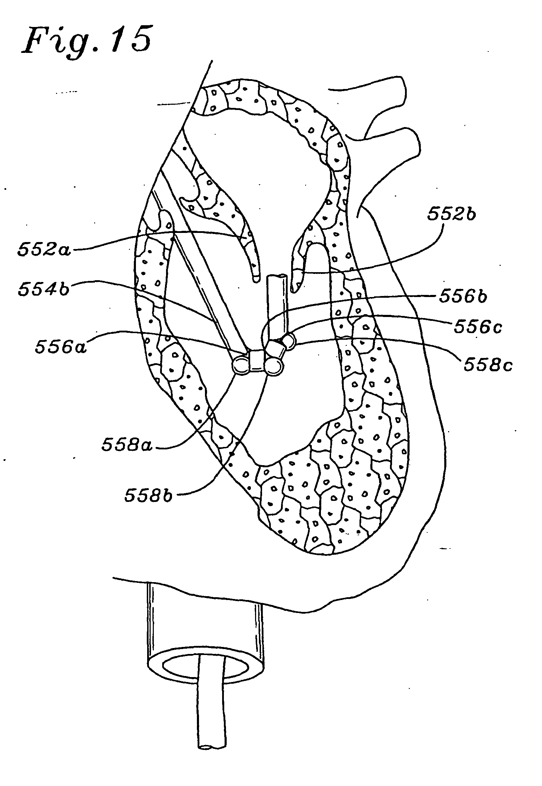

**Figure 15** shows a mitral valve procedure being performed by the present invention. Antegrade probe (not shown) is positioned proximate the arterial portion of the mitral tissue **552a** and **552b** by guidewires (not shown). The retrograde probe **554b** is positioned proximate the ventricular portion of the mitral tissue **552a** and **552b,** and is similarly directed by the guidewires. Retrograde probe **554b** further disposes at least one biasing joint containing at least one balloon which is connected to an inflation conduit (not shown) positioned within an internal lumen located in the probe **554b.** **Figure 15** shows a probe **554b** disposing 3 biasing joints **556a, 556b, and 556c,** each containing a steering balloon **558a, 558b, and 558c,** respectively. The probe **554b** is positioned proximate the tissue under repair. Once positioned, steering balloons **558a, 558b,** and **558c** are inflated thereby articulating the distal portion of the probe **554b** at an angle proximate the tissue.

### G. Sequential Tissue Stabilization

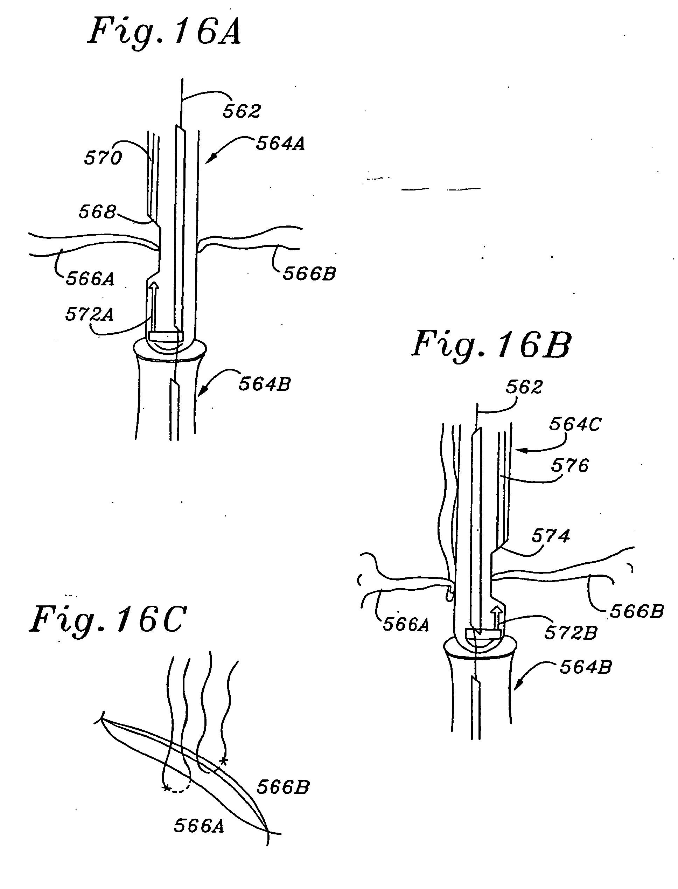

The present invention may be adapted to sequentially stabilize a portion of tissue and deploy a tissue fastening device therein. As shown in **Figure 16a****,** a first antegrade probe **564a** is advanced along at least one guidewire **562** to a position proximate the tissue to be repaired **566a** and **566b.** The first antegrade probe **564a** comprises a vacuum port **568** in fluid communication with a vacuum lumen **570** and a tissue fastening device **572a** located within the probe **564a.** The tissue fastening device **572a** may include fastener deployment mechanisms and fasteners disclosed above. A retrograde probe **564b,** which is used to position and stabilize the antegrade probe, is advanced along the at least one guidewire **562** to a position proximate the retrograde portion of the tissue. With the probes **564a** and **564b** positioned, a single portion of tissue **566a** is captured by the vacuum port **568** disposed on the first antegrade probe **564a.** A fastening device **572a** is deployed through the single portion of tissue **566a.** The first antegrade probe **564a** disengages the tissue **566a** and the retrograde probe **564b,** and is thereafter removed. **Figure 16b** shows a second antegrade probe **564c** comprising a vacuum port **574** in fluid communication with a vacuum lumen **576,** and a tissue fastening device **572b** located within the probe **564c** is advanced to a position proximate the tissue **566a** and **566b.** Like the first antegrade probe **564a,** the second antegrade probe **564c** is adapted to engage the retrograde probe **564b,** and deploy a tissue fastener. Once the probes are positioned, the vacuum port **574** disposed on the second antegrade probe **564c** captures tissue portion **566b.** A tissue fastener **572b** is deployed into the tissue. The second antegrade probe **564c** disengages the tissue **566b,** and the second antegrade probe **564c** and retrograde probe **564b** are removed. As shown in **Figure 16c****,** the tissue fastening device is joined, for example, by tying, thereby repairing the tissue. Like the previous embodiments the probes **564a, 564b,** and **564c** may include additional internal lumens.

## Claims

1. A system for performing a surgical procedure within a blood vessel, comprising:
at least one guidewire (12), said guidewire adapted to be inserted into a body vessel;
an ante grade probe (10a) having a distal portion, said antegrade probe comprising at least one antegrade guidewire lumen, said antegrade guidewire lumen terminating in at least one antegrade guidewire port (22);
a retrograde probe (106) having a distal portion, said retrograde probe comprising at least one retrograde guidewire lumen, said retrograde guidewire lumen terminating in at least one retrograde guidewire port (22), said at least one retrograde guidewire port aligned with said antegrade probe; and
at least one of said antegrade probe and said retrograde probe further comprising at least one lumen in addition to said retrograde and antegrade guidewire lumens;
wherein said antegrade probe (10a) and said retrograde probe (10b) are placed over said guidewire (12) so that said guidewire resides within said at least one antegrade guidewire port and said at least one retrograde guidewire port and wherein said at least one retrograde guidewire port is aligned with said at least one antegrade guidewire port.

2. The system of claim 1, further comprising a second guidewire (12) and wherein said antegrade probe comprises a first antegrade guidewire lumen terminating in a first antegrade guidewire port (22a) and a second antegrade guidewire lumen terminating in a second antegrade guidewire port (22b) and said retrograde probe comprises a first retrograde guidewire lumen terminating in a first retrograde guidewire port (22a) and a second retrograde guidewire lumen terminating in a second retrograde guidewire port (22b).

3. The system of claim 2, wherein said first guidewire resides within said first antegrade guidewire lumen and said first retrograde guidewire lumen and said second guidewire resides in said second antegrade guidewire lumen and said second retrograde guidewire lumen to align said distal portion of said antegrade probe with said distal portion of said retrograde probe.

4. The system of claim 1, wherein said antegrade probe and said retrograde probe are each engageable with one of the two pieces of tissue; to stabilize the tissue pieces.

5. The system of claim 4, wherein said antegrade probe and retrograde probe are mutually engageable with the two pieces of tissue to stabilize the tissue pieces interposed therebetween.

6. The system of claim 1, wherein said at least one lumen composes a vacuum lumen.

7. The system of claim 6, wherein said at least one vacuum lumen terminates in at least one vacuum port (24a, 24b) at said distal portion of said antegrade probe, thereby enabling the grasping and manipulation of tissue.

8. The system of claim 6, wherein said at least one vacuum lumen terminates in at least one vacuum port at said distal portion of said retrograde probe, thereby enabling the grasping and manipulation of tissue.

9. The system of claim 1, wherein at least one of said distal poison (20) of at least one of said antegrade probe and said retrograde probe is substantially perpendicular to said longitudinal axis of said antegrade or retrograde probe.

10. The system of claim 1, wherein said distal portion (33) of at least one said antegrade probe and said retrograde probe is tapered.

11. The system of claim 1, further comprising at least one tissue fastener (90) at the distal end of either said retrograde probe or said antegrade probe.

12. The system of claim 11, wherein said tissue fastener is a suture-based tissue fastener.

13. The system of claim 11, wherein said tissue fastener is a clip.

14. The system of clam 11, wherein said tissue fastener is a staple (280).

15. The system of claim 11, wherein the other one of said antegrade probe and retrograde probe further includes a tissue fastener receiver, said receiver providing cooperative stabilization of tissue while affixing said tissue fastener.

16. The system of claim 1, wherein a tissue fastener is positioned within said at least one lumen.

17. The system of claim 16, further comprising at least one tissue fastener at the distal end of either said retrograde probe or said antegrade probe.

18. The system of claim 17, wherein said tissue fastener is a needle (96) and suture (94).

19. The system or claim 1, wherein at least one of said antegrade probe distal portion and said retrograde probe distal portion disposes at least one deployable alignment mechanism (523).

20. The system of claim 19, the deployable alignment mechanism comprising:
at least two alignment arms flexibly attached to the distal portion of at least one of said antegrade probe and said retrograde probe;
a deployment conduit operably connected to said at least two alignment arms;
said deployment conduit attached to a deployment actuator;
said at least two alignment arms having a retracted position wherein said arms are located proximal to the distal portion of at least one of said antegrade probe and said retrograde probe;
said at least two alignment arms having a deployed position wherein said arms are extended radially from said distal portion of at least one of said antegrade probe and said retrograde probe; and
said retracted and deployed positions achieved through manipulation of said deployment actuator.

21. The system of claim 20, wherein the deployable alignment mechanism is positioned within said at least one lumen.

22. The system of claim 1, further comprising a steering mechanism located proximate to said distal portion of at least one of said antegrade probe and said retrograde probe.

23. The system of claim 22, the steering mechanism further comprising a steering conduit (536) attached to said distal portion of at least one of said antegrade probe and said retrograde probe, said steering conduit adapted for communication with an operator through one of said at least one antegrade lumen and said at least one retrograde lumen.

24. The system of claim 1, further comprising at least one echogenic member at or near the distal portion of one of said antegrade probe and said retrograde probe to enhance echo visualization.

25. The system of claim 1, further comprising a polymer coating wholly or selectively applied at or near the distal portion of one of said antegrade probe and said retrograde probe to enhance echo visualization.

## Patentansprüche

1. System zur Durchführung einer chirurgischen Prozedur innerhalb eines Blutgefäßes, umfassend:
Zumindest einen Führungsdraht (12), wobei besagter Führungsdraht angepasst ist, in ein Körpergefäß eingeschoben zu werden;
eine antegrade Sonde (10a) mit einem distalen Teil, wobei besagte antegrade Sonde zumindest ein antegrades Führungsdrahtlumen umfasst, wobei besagtes antegrade Führungsdrahtlumen in zumindest einer antegrade Führungsdrahtöffnung (22) endet;
eine retrograde Sonde (10b) mit einem distalen Teil, wobei besagte retrograde Sonde zumindest ein retrogrades Führungsdrahtlumen umfasst, besagtes retrograde Führungsdrahtlumen in zumindest einer retrograden Führungsdrahtöffnung (22) endet, besagte zumindest eine retrograde Führungsdrahtöffnung mit besagter antegraden Sonde fluchtet; und
Zumindest eine besagter antegraden Sonde und besagter retrograden Sonde weiter zumindest ein Lumen zusätzlich zu besagten retrograden und antegraden Führungsdrahtlumen umfassen;
Wobei besagte antegrade Sonde (10a) und besagte retrograde Sonde (10b) so über besagten Führungsdraht (12) platziert werden, dass besagter Führungsdraht in zumindest einer antegraden Führungsdrahtöffnung und besagter zumindest einen retrograden Führungsdrahtöffnung liegt und wobei besagte zumindest eine retrograde Führungsdrahtöffnung mit besagter zumindest einen antegraden Führungsdrahtöffnung fluchtet.

2. System nach Anspruch 1, das weiter einen zweiten Führungsdraht (12) umfasst und wobei besagte antegrade Sonde ein erstes antegrades Führungsdrahtlumen, das in einer ersten antegraden Führungsdrahtöffnung (22a) endet und ein zweites antegrades Führungsdrahtlumen, das in einer zweiten antegraden Führungsdrahtöffnung (22b) endet, umfasst und besagte retrograde Sonde ein erstes retrogrades Führungsdrahtlumen, das in einer ersten retrograden Führungsdrahtöffnung (22a) endet und ein zweites retrogrades Führungsdrahtlumen, das in einer zweiten retrograden Führungsdrahtöffnung (22b) endet, umfasst.

3. System nach Anspruch 2, wobei besagter erste Führungsdraht im besagten ersten antegraden Führungsdrahtlumen und besagten ersten retrograden Führungsdrahtlumen liegt und besagter zweite Führungsdraht im besagten zweiten antegraden Führungsdrahtlumen und besagten zweiten retrograden Führungsdrahtlumen liegt, um besagten distalen Teil besagter antegraden Sonde mit besagtem distalen Teil besagter retrograden Sonde zu fluchten.

4. System nach Anspruch 1, wobei besagte antegrade Sonde und besagte retrograde Sonde jeweils mit einem der zwei Gewebestücke in Eingriff bringbar sind, um die Gewebestücke zu stabilisieren.

5. System nach Anspruch 4, wobei besagte antegrade Sonde und besagte retrograde Sonde gegenseitig mit den zwei Gewebestücke in Eingriff bringbar sind, um die Gewebestücke zu stabilisieren.

6. System nach Anspruch 1, wobei besagtes zumindest eine Lumen ein Vakuumlumen umfasst.

7. System nach Anspruch 6, wobei besagtes zumindest eine Vakuumlumen in zumindest einer Vakuumöffnung (24a, 24b) an besagtem distalen Teil besagter antegraden Sonde endet, wodurch das Erfassen und Manipulieren von Gewebe ermöglicht wird.

8. System nach Anspruch 6, wobei besagtes zumindest eine Vakuumlumen in zumindest einer Vakuumöffnung (24a, 24b) an besagtem distalen Teil besagter retrograden Sonde endet, wodurch das Erfassen und Manipulieren von Gewebe ermöglicht wird.

9. System nach Anspruch 1; wobei zumindest eins besagten distalen Teils (20) von zumindest einer besagter antegraden Sonde und besagter retrograden Sonde im Wesentlichen senkrecht zur besagten Längsachse besagter antegraden oder retrograden Sonde ist.

10. System nach Anspruch 1, wobei besagtes distale Teil (33) von zumindest einer besagten antegraden Sonde und besagter retrograden Sonde konisch zulaufend ist.

11. System nach Anspruch 1, das weiter zumindest einen Gewebebefestiger (90) am distalen Ende entweder besagter retrograden Sonde oder besagter antegraden Sonde umfasst.

12. System nach Anspruch 11, wobei besagter Gewebebefestiger ist ein Befestiger auf Nahtbasis.

13. System nach Anspruch 11, wobei besagter Gewebebefestiger ist ein Klip ist.

14. System nach Anspruch 1, wobei besagter Gewebebefestiger eine Klammer (280) ist.

15. System nach Anspruch 11, wobei die Andere besagter antegrader Sonde und retrograder Sonde weiter einen Empfänger für den Gewebebefestiger umfasst, wobei besagter Empfänger kooperative Stabilisierung des Gewebes, während des Anbringens besagten Gewebebefestigers, bereitstellt.

16. System nach Anspruch 1, wobei ein Gewebebefestiger im besagten zumindest einem Lumen positioniert wird.

17. System nach Anspruch 16, das weiter zumindest einen Gewebebefestiger am distalen Ende entweder besagter retrograden Sonde oder besagter antegraden Sonde umfasst.

18. System nach Anspruch 17, wobei besagter Gewebebefestiger ist eine Nadel (96) und eine Naht (94) ist.

19. System nach Anspruch 1, wobei zumindest ein distaler Teil besagter antegraden Sonde und ein distaler Teil besagter retrograden Sonde zumindest einen ausschwenkbaren Ausrichtmechanismus (523) anwendet.

20. System nach Anspruch 19, wobei der ausschwenkbare Ausrichtmechanismus umfasst:
Zumindest zwei Ausrichtarme, die flexibel am distalen Teil zumindest einer der besagten antegraden Sonde und besagten retrograden Sonde befestigt sind;
Ein Einsatzrohr, das betriebsfähig mit besagten zumindest zwei Ausrichtarmen verbunden ist;
besagtes Einsatzrohr, das an einem Einsatzaktuator befestigt ist;
besagte zumindest zwei Ausrichtarme mit einer eingefahrenen Position, wobei sich besagte Arme proximal zum distalen Teil zumindest einer der besagten antegraden Sonde und der besagten retrograden Sonde befinden;
besagte zumindest zwei Ausrichtarme mit einer ausgeschwenkten Position, wobei besagte Arme radial vom besagten distalen Teil zumindest einer der besagten antegraden Sonde und der besagten retrograden Sonde ausgefahren sind; und
besagte eingefahrenen und ausgeschwenkten Positionen durch Manipulation des besagten Einsatzaktuators erzielt werden.

21. System nach Anspruch 20, wobei der ausschwenkbare Ausrichtmechanismus im besagten zumindest einem Lumen positioniert wird.

22. System nach Anspruch 1, das weiter einen Steuerungsmechanismus umfasst, der sich nahe dem besagten distalen Teil der zumindest einen besagter antegraden Sonde und besagten retrograden Sonde befindet.

23. System nach Anspruch 22, wobei der Steuerungsmechanismus weiter ein Steuerrohr (536) umfasst, das am besagten distalen Teil der zumindest einen der besagten antegraden Sonde und der besagten retrograden Probe befestigt ist, wobei besagtes Steuerrohr zur Kommunikation mit einem Bediener durch eines des besagten zumindest einen antegraden Lumens und besagten zumindest einen retrograden Lumens angepasst ist.

24. System nach Anspruch 1, das weiter zumindest ein Echo gebendes Bauelement am oder nahe dem distalen Teil einer der besagten antegraden Sonde und der besagten retrograden Sonde umfasst, um Echovisualisierung zu verbessern.

25. System nach Anspruch 1, das weiter eine Polymerbeschichtung umfasst, die gänzlich oder selektiv am oder nahe dem distalen Teil einer der besagten antegraden Sonde aufgebracht ist, um Echovisualisierung zu verbessern.

## Revendications

1. Un système de réalisation d'une procédure chirurgicale dans un vaisseau sanguin comprenant :
au moins un fil guide (12), ledit fil guide étant adapté pour être inséré dans un vaisseau corporel ;
une sonde de mise en place par voie antégrade (10a) possédant une partie distale, ladite sonde de mise en place par voie antégrade comprenant au moins une lumière d'introduction du fil guide de l'abord antégrade, ladite lumière d'introduction du fil de l'abord antégrade se terminant dans au moins un port d'introduction du fil guide de l'abord antégrade (22) ;
une sonde de mise en place par voie rétrograde (10b) possédant une partie distale, ladite sonde de mise en place par voie rétrograde comprenant au moins une lumière d'introduction du fil guide de l'abord rétrograde, ladite lumière d'introduction du fil guide de l'abord rétrograde se terminant dans au moins un port d'introduction du fil guide de l'abord rétrograde (22), ledit au moins un port d'introduction du fil guide de l'abord rétrograde étant aligné avec ladite sonde de mise en place par voie antégrade ; et
au moins une de ladite sonde de mise en place par voie antégrade et de ladite sonde de mise en place par voie rétrograde comprenant en outre au moins une lumière en plus desdites lumières d'introduction du fil guide de l'abord rétrograde et de l'abord antégrade ;
ladite sonde de mise en place par voie antégrade (10a) et ladite sonde de mise en place par voie rétrograde (10b) étant placées par-dessus ledit fil guide (12) de sorte que le fil guide réside au sein dudit au moins un port d'introduction du fil guide de l'abord antégrade et dudit au moins un port d'introduction du fil guide de l'abord rétrograde et ledit au moins un port d'introduction du fil guide de l'abord rétrograde étant aligné avec ledit au moins un port d'introduction du fil guide de l'abord antégrade.

2. Système de la revendication 1, comprenant en outre un deuxième fil guide (12) et la sonde de mise en place par voie antégrade comprenant une première lumière d'introduction du fil guide de l'abord antégrade se terminant dans un premier port d'introduction du fil guide de l'abord antégrade (22a) et une deuxième lumière d'introduction du fil guide de l'abord antégrade se terminant dans un deuxième port d'introduction du fil guide de l'abord antégrade (22b) et ladite sonde de mise en place par voie rétrograde comprenant une première lumière d'introduction du fil guide de l'abord rétrograde se terminant dans un premier port d'introduction du fil guide de l'abord rétrograde (22a) et une deuxième lumière d'introduction du fil guide de l'abord rétrograde se terminant dans un deuxième port d'introduction du fil guide de l'abord rétrograde (22b).

3. Système de la revendication 2, ledit premier fil guide résidant au sein de ladite lumière d'introduction du fil guide de l'abord antégrade et ladite première lumière d'introduction du fil guide de l'abord rétrograde et ledit deuxième fil guide résidant dans ladite deuxième lumière d'introduction du fil guide de l'abord antégrade et ladite deuxième lumière d'introduction du fil guide de l'abord rétrograde de manière à aligner ladite partie distale de ladite sonde de mise en place par voie antégrade avec ladite partie distale de ladite sonde de mise en place par voie rétrograde.

4. Système de la revendication 1, ladite sonde de mise en place par voie antégrade et ladite sonde de mise en place par voie rétrograde étant chacune engageable avec l'une des deux pièces de tissu de sorte à stabiliser les pièces de tissu.

5. Système de la revendication 4, ladite sonde de mise en place par voie antégrade et la sonde de mise en place par voie rétrograde étant mutuellement engageables avec les deux pièces de tissu de sorte à stabiliser les pièces de tissu interposées entre elles.

6. Système de la revendication 1, ladite au moins une lumière comprenant une lumière sous vide.

7. Système de la revendication 6, ladite au moins une lumière d'aspiration se terminant dans au moins un port de vide (24a, 24b) à ladite partie distale de ladite sonde de mise en place par voie antégrade, permettant ainsi la préhension et la manipulation du tissu.

8. Système de la revendication 6, ladite au moins une lumière d'aspiration se terminant dans au moins un port de vide à ladite partie distale de ladite sonde de mise en place par voie rétrograde, permettant ainsi la préhension et la manipulation du tissu.

9. Système de la revendication 1, au moins une de ladite partie distale (20) d'au moins une de ladite sonde de mise en place par voie antégrade et de ladite sonde de mise en place par voie rétrograde étant sensiblement perpendiculaire audit axe longitudinal de ladite sonde de mise en place par voie antégrade ou rétrograde.

10. Système de la revendication 1, ladite partie distale (33) d'au moins une de ladite sonde de mise en place par voie antégrade et de ladite sonde de mise en place par voie rétrograde étant effilée.

11. Système de la revendication 1, comprenant en outre au moins une attache tissulaire (90) à l'extrémité distale soit de ladite sonde de mise en place par voie rétrograde, soit de ladite sonde de mise en place par voie antégrade.

12. Système de la revendication 11, l'attache tissulaire étant une attache tissulaire à base de suture.

13. Système de la revendication 11, ladite attache tissulaire étant un clip.

14. Système de la revendication 11, ladite attache tissulaire étant une agrafe (280).

15. Système selon la revendication 11, l'autre desdites sonde de mise en place par voie antégrade et sonde de mise en place par voie rétrograde comprenant un receveur d'attache tissulaire, ledit receveur assurant une stabilisation coopérative du tissu lors de l'apposition de ladite attache tissulaire.

16. Système de la revendication 1, l'attache tissulaire étant positionnée au sein de ladite au moins une lumière.

17. Système de la revendication 16, comprenant en outre une attache tissulaire à l'extrémité distale soit de ladite sonde de mise en place par voie rétrograde, soit de ladite sonde de mise en place par voie antégrade.

18. Système de la revendication 17, ladite attache tissulaire étant une aiguille (96) et une suture (94).

19. Système de la revendication 1, au moins une de ladite partie distale de la sonde de mise en place par voie antégrade et de ladite partie distale de la sonde de mise en place par voie rétrograde disposant d'au moins un mécanisme d'alignement déployable (523).

20. Système de la revendication 19, le mécanisme d'alignement déployable comprenant :
au moins deux bras d'alignement flexiblement attachés à la partie distale d'au moins une desdites sonde de mise en place par voie antégrade et sonde de mise en place par voie rétrograde ;
un conduit de déploiement opérativement connecté auxdits au moins deux bras d'alignement ;
ledit conduit de déploiement étant attaché à l'actionneur de déploiement ;
lesdits au moins deux bras d'alignement ayant une position rétractée, lesdits bras étant situés à un emplacement proximal à la partie distale d'au moins une de ladite sonde de mise en place par voie antégrade et de ladite sonde de mise en place par voie rétrograde ;
lesdits au moins deux bras d'alignement ayant une position déployée selon laquelle lesdits bras sont prolongés radialement depuis ladite partie distale d'au moins une de ladite sonde de mise en place par voie antégrade et de ladite sonde de mise en place par voie rétrograde ; et
lesdites positions rétractées et déployées étant obtenues par manipulation dudit actionneur de déploiement.

21. Système de la revendication 20, selon lequel le mécanisme d'alignement déployable est positionné au sein de ladite au moins une lumière.

22. Système de la revendication 1, comprenant en outre un mécanisme de direction situé à proximité de ladite partie distale d'au moins une de ladite sonde de mise en place par voie antégrade et de ladite sonde de mise en place par voie rétrograde.

23. Système de la revendication 22, le mécanisme de direction comprenant en outre un conduit de direction (536) attaché à ladite partie distale d'au moins une de ladite sonde de mise en place par voie antégrade et de ladite sonde de mise en place par voie rétrograde, ledit conduit de direction étant adapté pour une communication avec un opérateur par le biais de l'une de ladite au moins une lumière de mise en place par voie antégrade et de ladite au moins une lumière de mise en place par voie rétrograde.

24. Système de la revendication 1 comprenant en outre au moins un organe échogénique à ou à proximité de la partie distale de l'une de ladite sonde de mise en place par voie antégrade et de ladite sonde de mise en place par voie rétrograde de manière à améliorer la visualisation des échos.

25. Système de la revendication 1, comprenant en outre un revêtement de polymère entièrement ou sélectivement appliqué sur ou à proximité de l'une des ladite sonde de mise en place par voie antégrade et de ladite sonde de mise en place par voie rétrograde de manière à améliorer la visualisation des échos.
